# EUROPEAN PATENT SPECIFICATION

(11) **EP 3 863 598 B1**
(45) Date of publication and mention of the grant of the patent: **29.04.2026**
(21) Application number: 19779036.3
(22) Date of filing: 04.10.2019
(51) Int. Cl.: A61K 8/67, A61K 8/86, A61Q 5/00, A61Q 19/00

(54) **TOPICAL COMPOSITION**
TOPISCHE ZUSAMMENSETZUNG
COMPOSITION TOPIQUE

(30) Priority: 09.10.2018 EP 18199403
(43) Date of publication of application: 18.08.2021
(73) Proprietor: DSM IP Assets B.V., 6221 BE Maastricht (NL)
(72) Inventor: MENDROK-EDINGER, Christine, 4303 Kaiseraugst (CH)
(74) Representative: dsm-firmenich IP
(86) International application number: PCT/EP2019/076866
(87) International publication number: WO 2020/074375

(56) References cited:
- EP-A1- 2 335 697
- US-A- 2 955 115
- US-A1- 2003 190 337

## Description

The present as defined in the appended claims invention is concerned with the use of a PEG-emulsifier, optionally in combination with hyaluronic acid or a salt thereof for suppressing discoloration in topical compositions comprising vitamin B6 or a derivative thereof. Furthermore, the invention relates to a method for reducing discoloration of topical compositions containing vitamin B6 or a derivative thereof, wherein said method comprises preparing a topical composition comprising vitamin B6 or a derivative thereof, a PEG-emulsifier and optionally hyaluronic acid or a salt thereof and a cosmetically acceptable carrier.

Vitamin B6 respectively derivatives thereof such as pyridoxine hydrochloride is widely used in the formulation of bath products, soaps and detergents, cleansing products, skin care products and hair care products. However, topical compositions such as in particular O/W emulsions containing vitamin B6 respectively a derivative thereof, such as in particular pyridoxine hydrochloride tend to discolor upon storage which is highly unwanted as it leads to an unpleasant optical appearance of the respective product.

In accordance with the present invention it has surprisingly been found that the discoloration of topical compositions containing vitamin B6 respectively derivatives thereof such as in particular pyridoxine hydrochloride can be suppressed by using a PEG-emulsifier, more preferably in combination with hyaluronic acid or a salt thereof, as the addition of sodium hyaluronate even further reduces the discoloration.

Thus, in one aspect, the present invention is concerned with the use of a PEG-emulsifier, optionally in combination with hyaluronic acid or a salt thereof for suppressing discoloration in topical compositions comprising Vitamin B6 or a derivative thereof.

In another embodiment the invention is concerned with a method for reducing discoloration of topical compositions containing Vitamin B6 or a derivative thereof, said method comprising preparing a topical composition comprising Vitamin B6 respectively a derivative thereof, a PEG-emulsifier, optionally hyaluronic acid or a salt thereof and a cosmetically acceptable carrier, and optionally storing the respective composition for at least 2, preferably for at least 3 months.

In a further aspect the invention relates to the use of a combination of Vitamin B6 or a derivative thereof and a PEG-emulsifier, and optionally hyaluronic acid or a salt thereof, for the preparation of storage stable topical composition. These compositions exhibit an excellent storage stability in view of preventing/suppressing discoloration.

The term Vitamin B6 and derivatives thereof refers in particular to pyridoxine hydrochloride [58-56-0], pyridoxal [CAS-Nr. 66-72-8] and pyridoxamin [CAS-Nr. 85-87-0]. Particularly preferred in all embodiments of the present invention is the use of pyridoxine hydrochloride also known as vitamin B6 hydrochloride or vitamin B6 which is e.g. commercially available as Pyridoxine hydrochloride or Pyridoxine Hydrochloride 98 DC by DSM Nutritional Products AG, (4303 Kaiseraugst, Switzerland).

The amount of Vitamin B6 or a derivative thereof such as in particular pyridoxine hydrochloride in the compositions according to the present invention is selected in the range of 0.02 to 6 wt. %, preferably in the range of 0.05 to 4 wt. %, most preferably in the range of 0.1 to 3 wt. %, based on the total weight of the composition.

The term PEG-emulsifier refers to polyethylene glycol based emulsifiers which are well known to a person skilled in the art. Such emulsifiers comprise at least one polyethylene glycol-containing (PEG) emulsifier, optinally in admixture with a suitable co-emulsifier such as e.g. PEG-100 stearate, PEG-25-hydrated ricinus oil, PEG-54-hydrated ricinus oil and/ or PEG-6 caprylic acid/caprinic acid glycerides, PEG-9-Stearate, PEG-20 Stearate, PEG-30-Stearate, PEG-40-stearate, PEG-40-Sorbitanperoleat, glycerylstearat in combination with PEG-30 stearate, PEG-40-stearate, PEG-22-dodecyl glycol Copolymer, polyglyceryl-2-PEG-4-stearat, PEG-20-stearat, steareth-2, steareth-21, steareth-100, steareth-2 in combination with PEG-8 distearate, PEG-45/ dodecylglycol-copolymer, methoxy-PEG-22/dodecylglycol-copolymer, PEG-40-sorbitanperoleat, PEG-40-sorbitanperisostearats, PEG-20-glycerylstearats, PEG-20-glycerylstearats, PEG-8-bee wax, PEG-20-methylglucosesesquistearat, cetylstearylalcohol in combination with PEG-20 stearate, PEG-30-stearate, PEG-40-stearate and/ or PEG-100-stearate, ceteth-2, ceteth-20, ceteareth-12, laureth-4, oleth-2, PEG-40 hydrogenated castor oil, PEG-8 cocoate, PEG-2 diisostearate, PEG-4 dialuarate, PEG-10 glyceryl stearate, PEG/PPG-18/6 Dimethicone, Bis-PEG/PPG-18/6 Dimethicone and PEG-20 Cocoate. Particularly preferred in all embodiments are PEG-emulsifiers which contain at least one PEG fatty acid, such as PEG-100 stearate, PEG-20 Cocoate and/ or Steareth-21.

The most preferred PEG-emulsifier in all embodiments of the present invention is PEG-100 stearate, which is even more preferably used in combination with glyceryl stearate, which mixture is e.g. commercially available as Arlacel 165 [CAS No: 31566-31-1, 9004-99-3] by Croda.

It is well understood that the topical compositions according to the present invention advantageously do not contain a cetyl phosphate emulsifier, even more preferably no anionic emulsifier. Most preferably in all embodiments of the present invention, the topical compositions only comprise a PEG-emulsifier (as sole emulsifier), optionally in admixture with at least one fatty alcohol co-emulsifier such as in particular cetyl alcohol and/ or cetearyl alcohol.

Thus, in a preferred embodiment, the topical compositions comprise, next to the PEG-emulsifier no further emulsifier.

The amount of the PEG-emulsifier in the compositions according to the present invention is selected in the range of 0.02 to 6 wt.-%, preferably in the range of 0.05 to 4 wt.-%, most preferably in the range of 0.1 to 3 wt.-%, based on the total weight of the composition.

If present, the amount of glyceryl stearate in the compositions according to the present invention is advantageously selected in the range of 0.02 to 5 wt.-%, preferably in the range of 0.1 to 4 wt.-%, most preferably in the range of 0.25 to 3 wt.-%, such as in the range of 0.25 to 2 wt.-%, based on the total weight of the composition.

If used in combination, preferably the ratio (w/w) of PEG-100 stearate to glyceryl stearate is selected in the range of 70:30 to 30:70, more preferably in the range of 60:40 to 40:60, such as in the range of about 55:45.

Hyaluronic acid also called hyaluronan, is an anionic, non-sulfated glycosaminoglycan which is widely used in cosmetic applications due to its excellent moisturizing properties. It is either used as free acid or in the form of a cosmetically acceptable salt thereof such as in particular as sodium salt (i.e. as sodium hyaluronate, CAS No 9067-32-7).

Particularly suitable in all embodiments of the present invention is the use of Hyasol PF which is commercially available from DSM Nutritional Products Ltd and which is an aqueous solution of a sodium hyaluronate with a molecular mass of the hyaluron polymer of about 1.6 MDa.

Further suitable hyaluronic acids according to the present invention include AEC Hyaluronic Acid, AEC Sodium Hyaluronate which are commercially available from A&E Connock, SpecKare HA which is commercially available from Spec-Chem Industry Inc., Bashyal Poudre which is commercially available from Givaudan Active Beauty, HyaCare Poudre which is commercially available from Evonic Nutrition & Care, Hyaluronate F100 which is commercially available from HTL, Hyaluronic Acid 1% 5P Poudre which is commercially available from CEP Solabia Group, Hyaluronsan HA-Q Poudre which is commercially available from Kewpie Corporation as well as Sodium Hyaluronate 1% Poudre which is commercially available from Tri-K Industries.

Preferably, the amount of the hyaluronic acid respectively a salt thereof in the compositions according to the present invention is selected in the range of 0.01 to 5 wt.-%, preferably in the range of 0.02 to 3 wt.-%, most preferably in the range of 0.05 to 2 wt.-%, based on the total weight of the composition.

In another aspect the present invention is concerned with storage stable topical compositions comprising Vitamin B6 respectively a derivative thereof such as pyridoxine hydrochloride and a PEG-emulsifier, and optionally hyaluronic acid and a cosmetically acceptable carrier. These compositions exhibit an excellent storage stability in view of preventing/suppressing discoloration.

The term "topical composition" as used herein refers in particular to cosmetic compositions that can be topically applied to mammalian keratinous tissue such as e.g. human skin or hair, particularly human skin.

The term "cosmetic composition" as used in the present application refers to cosmetic compositions as defined under the heading "Kosmetika" in Römpp Lexikon Chemie, 10th edition 1997, Georg Thieme Verlag Stuttgart, New York as well as to cosmetic preparations as disclosed in A. Domsch, "Cosmetic Preparations", Verlag für chemische Industrie (ed. H. Ziolkowsky), 4th edition, 1992.

The term 'cosmetically acceptable carrier' (also referred to herein as carrier) refers to all vehicles/ carriers conventionally used in cosmetic compositions, i.e. which are suitable for topical application to the keratinous tissue, have good aesthetic properties, are compatible with the actives present in the composition, and will not cause any unreasonable safety or toxicity concerns. Such carriers are well-known to one of ordinary skill in the art.

The exact amount of carrier will depend upon the actual level of the active ingredients and of any other optional ingredients that one of ordinary skill in the art would classify as distinct from the carrier (e.g., other active ingredients).

In an advantageous embodiment, the compositions according to the present invention comprise from about 50% to about 99%, preferably from about 60% to about 98%, more preferably from about 70% to about 98%, such as in particular from about 80% to about 95% of a carrier, based on the total weight of the composition.

In an advantageous embodiment, the carrier consists furthermore of at least 40 wt.-%, more preferably of at least 50 wt.-%, most preferably of at least 55 wt.-% of water, such as in particular of about 55 to about 90 wt.-% of water.

The topical compositions according to the present invention are generally in the form of an emulsion or micro emulsion (in particular of O/W- or W/O-type), PIT-emulsion, multiple emulsion (e. g. O/W/O- or W/O/W-type), pickering emulsion, hydrogel, alcoholic gel, lipogel, one- or multiphase solution or vesicular dispersion. which can also be applied by pens, as masks or as sprays. If the topical preparation is or comprises an emulsion it can also contain one or more anionic, nonionic, cationic or amphoteric surfactant(s).

The compositions according to the present invention are advantageously in the form of an oil-in-water (O/W) emulsion comprising an oily phase dispersed in an aqueous phase in the presence of the PEG-emulsifier. The preparation of such O/W emulsions is well known to a person skilled in the art.

The amount of the oily phase present in such emulsions is preferably at least 10 wt.-%, such as in the range of 10 to 60 wt.-%, preferably in the range of 15 to 50 wt.-%, most preferably in the range of 15 to 40 wt.-%, based on the total weight of the composition.

The oil phase of the O/W emulsions according to the invention preferably comprises oils selected from butylenglykoldicaprylat/-dicaprat, propylenglykoldicaprylat/-dicaprat, dicaprylylether, C₁₂₋₁₅-Alkylbenzoat, C₁₈₋₃₈-fatty acid triglyceride, dibutyladipate, cyclomethicone, dimethicone, 2-phenylethylbenzoat, isopropyl lauroyl sarkosinate, , caprylic/ capric triglyceride as well as mixtures thereof.

Topical compositions in accordance with the invention can be in the form of a liquid, lotion, a thickened lotion, a gel, a cream, a milk, an ointment, a paste, a powder, a make-up, or a solid tube stick and can be optionally be packaged as an aerosol and can be provided in the form of a mousse such as a aerosol mousse, a foam or a spray foam, a spray, a stick.

The topical cosmetic compositions of the invention can also contain usual cosmetic adjuvants and additives such as e.g. further UV filter substances, preservatives, antioxidants, fatty substances, oils, water, alcohols, polyols, organic solvents, electrolytes, silicones, thickeners, film forming agents, softeners, emulsifiers, complexing agents, antifoaming agents, moisturizers, aesthetic components such as fragrances, surfactants, fillers, sequestering agents, anionic, cationic, nonionic or amphoteric polymers or mixtures thereof, propellants, acidifying or basifying agents, dyes, colorings/colorants, abrasives, absorbents, essential oils, skin sensates, astringents, antifoaming agents, pigments or nanopigments, cosmetically active ingredients or any other ingredients, carriers and/or excipients or diluents conventionally formulated into cosmetic compositions.

Examples of cosmetic excipients, diluents, adjuvants, additives as well as active ingredients commonly used in the skin care industry which are suitable for use in the cosmetic compositions of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The necessary amounts of the active ingredients as well as the excipients, diluents, adjuvants, additives etc. can, based on the desired product form and application, easily be determined by the skilled person. The additional ingredients can either be added to the oily phase, the aqueous phase or separately as deemed appropriate.

The cosmetically active ingredients useful herein can in some instances provide more than one benefit or operate via more than one mode of action.

Preferred topical compositions according to the invention further comprise one or several antioxidant(s). Suitable antioxidants for the incorporation into the topical compositions according to the invention are all antioxidant suitable for cosmetic applications. Particular preferred are water soluble antioxidants such as vitamins such as e.g. ascorbic acid as well as derivatives thereof such as e.g. ascorbyl phosphate such as Stay C (sodium ascorbyl monophosphate) from DSM Nutritional Products Ltd.

Further preferred antioxidants are BHT, vitamin E and derivatives thereof as well as vitamin A and derivatives thereof.

The amount of antioxidant (one or several) in the topical compositions according to the invention is preferably selected in the range of about 0.001 to 30 wt.-%, in particular in the range of about 0.05 to 20 wt.-%, most particular in the range of about 0.1 to 10 wt.-%, with respect to the total amount of the topical composition.

If a vitamin E derivative is used in the topical compositions according to the invention preferably tocopheryl acetate is used. Tocopheryl acetate may be present in the topical preparations in an amount from about 0.05 to 25 wt.-%, in particular 0.5 to 5 wt.-%. Another vitamin E derivative of interest is tocopheryl linoleate. Tocopheryl linoleate may be present in the skin care composition in an amount from about 0.05 to 25 wt.-% in particular 0.5 to 5 wt.-%.

Vitamin A and/or its derivatives in particular retinoid derivatives such as retinyl palmitate or retinyl propionate is preferably used in the topical preparations according to the invention in an amount of 0.01 to 5 wt.-%, in particular 0.01 to 0.3 wt.-%.

Preferably, the topical compositions according to the invention further comprise at least one fatty alcohol (co-emulsifier), such as in particular cetyl alcohol, cetearyl alcohol and/ or behenyl alcohol. The total amount of one or several fatty alcohols on the topical compositions according to the invention is preferably selected in the range of about 0.1 to 10.0 wt.-%, in particular in the range of about 0.5 to 6.0 wt.-% with respect to the total weight of the topical composition.

Suitable cosmetically active ingredients according to the invention encompass agents suitable for skin lightening; tanning prevention; self tanning; treatment of hyperpigmentation; preventing or reducing acne; treatment of wrinkles, lines, atrophy and/or inflammation; treatment of cellulites (e.g. phytanic acid), firming, energizing, skin soothing, as well as agents to improve skin elasticity and skin barrier.

The cosmetically active ingredients useful herein can in some instances provide more than one benefit or operate via more than one mode of action.

Preferred examples of cosmetically active ingredients for the incorporation into topical compositions according to the invention encompass vitamin B₆, vitamin B₁₂, biotin, co-enzyme Q10, EGCG, alpha-liponic acid, phytoen, hydroxytyrosol and/or olive extract, shea butter, algae extract, cocoa butter, aloe extract, jojoba oil, echinacea extract, chamomile extract, alpha-glucosylrutin, carnitin, carnosine, natural and/ or synthetic isoflavanoids, creatin, taurin, alanine, glycyrrhetinic acid, glycyryca glabra and/ or glycyrrhiza inflata.

The cosmetically active ingredient is typically included in an amount of at least 0.001 wt. % based on the total weight of the topical preparation. Generally, an amount of about 0.001 wt. % to about 30 wt. %, preferably from about 0.001 wt. % to about 10 wt. % of an additional cosmetically active agent is used. Further preferred the topical compositions according to the invention further comprise a moisturizer. Moisturizers are chemical agents specially designed to make the external layers of the skin (epidermis) softer and more pliable by increasing its hydration (water content) which can be determined e.g. by measuring the transepidermal water loss (TEWL). Suitable moisturizer according to the invention are for example glycerin, lactic acid and/ or lactate such as in particular sodium lactate, butyleneglycol, propyleneglycol, biosaccaride gum-1, glycine soja, ethylhexyloxyglycerine, pyrrolidoncarbonic acid, ssaccahride isomerate and/or urea.

Preferably, the topical compositions according to the invention comprise a thickener in particular if the topical composition is in the form of an emulsion to assist in making the consistency of a product suitable. Preferred thickeners are aluminiumsilicates, xanthan gum, hydroxypropylmethylcellulose, hydroxyethylcellulose, polyacrylates such as carbopole^{®} (e.g. Carbopole 980, 981, 1382, 2984, 5984) or mixtures thereof. Further preferred thickeners encompass acrylate/C₁₀₋₃₀ alkyl acrylate copolymers (such as e.g. Pemulen TR 1, Pemulen TR 2, Carbopol 1328 by. NOVEON) as well as Aristoflex AVC (INCI: Ammonium Acryloyldimethyltaurate/VP Copolymer).

The cosmetic compositions according to the present invention advantageously comprise a preservative. Particular suitable preservatives in all embodiments of the present invention are hydroxyacetophenone, phenoxyethanol and ethylhexylglycerin as well as mixtures thereof. When present, the preservative is preferably used in an amount of 0.1 to 2 wt.-%, more preferably in an amount of 0.5 to 1.5 wt.-%, based on the total weight of the composition.

The compositions according to the invention in general have a pH in the range of 3 to 10, preferably a pH in the range of 4 to 8 and most preferably a pH in the range of 5 to 8. The pH can easily be adjusted as desired with suitable acids, such as e.g. citric acid, or bases, such as sodium hydroxide (e.g. as aqueous solution), triethanolamine (TEA Care), Tromethamine (Trizma Base) and Aminomethyl Propanol (AMP-Ultra PC 2000), according to standard methods in the art.

The amount of the compositions to be applied to the skin is not critical and can easily be adjusted by a person skilled in the art. Preferably the amount is selected in the range of 0.1 to 3 mg/ cm² skin, such as preferably in the range of 0.1 to 2 mg/ cm² skin and most preferably in the range of 0.5 to 2 mg / cm² skin.

The following examples are provided to further illustrate the compositions and effects of the present invention.

### Example 1

The formulations as outlined in table 1 have been prepared and stored for 3 months at 40°C. As reference the same composition comprising a cetyl phosphate emulsifier has been prepared. The colour stability was assessed by measuring the b values (L* a* b* values/ CIELAB system) at t0 and after 3 months storage, which is an indicator of the discoloration of the sample (the higher the b-value, the higher the discoloration).

**Table 1**

| INCI | Ref. 1 Wt% | Inv. 1 Wt% | Inv. 2 Wt% |
|---|---|---|---|
| PEG-100 Stearate, Glyceryl Stearate (Arlacel 165) | | 2.0 | 2.0 |
| Potassium Cetyl Phosphate (Amphisol K) | 2.0 | | |
| Cetyl Alcohol | 3.0 | 3.0 | 3.0 |
| Cetearyl Alcohol | 2.0 | 2.0 | 2.0 |
| Dimethicone | 3.0 | 3.0 | 3.0 |
| Caprylic/Capric Triglyceride | 10.0 | 10.0 | 10.0 |
| Glycerin | 3.0 | 3.0 | 3.0 |
| Xanthan Gum | 0.3 | 0.3 | 0.3 |
| Phenoxyethanol, Ethylhexylglycerin | 1.0 | 1.0 | 1.0 |
| Pyridoxin HCl | 1.0 | 1.0 | 1.0 |
| Hyaluronic Acid (Hyasol PF) | | | 3.0 |

| Aqua | Ad 100 | Ad 100 | Ad 100 |
|---|---|---|---|
| b-value initial | -0.90 | -0.70 | -0.72 |
| b-value after 3 months/ 40°C | +0.46 | -0.40 | -0.37 |

Surprisingly, the use of a PEG-emulsifier solved the problem of discoloration, while the addition of sodium hyaluronate even ameliorated the effect. The color of the formulation stayed cream-white over the whole storage time while the formulation comprising a cetyl phosphate emulsifier turned yellow.

### Example 2:

The formulations as outlined in table 2 and 3 have been prepared and stored for 6 weeks on a window board. The reference comprised a cetyl phosphate emulsifier instead of a PEG-emulsifier. Table 2 outline examples with 2 wt.-% of emulsifiers and 0.5 wt.-% Vitamin B6 whereas table 3 outline examples with 2 wt.-% of emulsifiers and 0.1 wt.-% of vitamin B6.

The colour stability was assessed by measuring the L* a* b* values at t0 and after 6 weeks storage. Then the ΔE values after 6 weeks storage versus t0 have been calculated.

The L* a* b* measurement determines a possible color change of formulations. The L* a* b* system is also referred to as the CIELAB system. It can be visualized as a cylindrical coordinate system in which the axis of the cylinder is the lightness variable L*, ranging from 0% to 100%, and the radii are the chromaticity variables a* and b*. Variable a* is the green (negative) to red (positive) axis, and variable b* is the blue (negative) to yellow (positive) axis.

Based on these values, the ΔE values can be calculated. The ΔE value reflects the difference of the overall color of a formulation over storage time. The higher the ΔE value the stronger the difference of color.

**Table 2:**

| INCI | Ref. 1 Wt% | Inv. 1 Wt% | Inv. 2 Wt% | Inv. 3 Wt% | Inv. 4 Wt% | Inv. 5 Wt% | Inv. 6 Wt% |
|---|---|---|---|---|---|---|---|
| Potassium Cetyl Phosphate (Amphisol K) | 2.0 | | | | | | |
| PEG-100 Stearate, Glyceryl Stearate (Arlacel 165) | | 2.0 | 2.0 | | | | |
| Steareth-21 (Eumulgin S21) | | | | 2.0 | 2.0 | | |
| PEG-20 Cocoate (Hydriol KP 10) | | | | | | 2.0 | 2.0 |
| Cetyl Alcohol | 3.0 | 3.0 | 3.0 | 3.0 | 3.0 | 3.0 | 3.0 |
| Cetearyl Alcohol | 2.0 | 2.0 | 2.0 | 2.0 | 2.0 | 2.0 | 2.0 |
| Dimethicone | 3.0 | 3.0 | 3.0 | 3.0 | 3.0 | 3.0 | 3.0 |
| Caprylic/Capric Triglyceride | 10.0 | 10.0 | 10.0 | 10.0 | 10.0 | 10.0 | 10.0 |
| Glycerin | 3.0 | 3.0 | 3.0 | 3.0 | 3.0 | 3.0 | 3.0 |
| Xanthan Gum | 0.3 | 0.3 | 0.3 | 0.3 | 0.3 | 0.3 | 0.3 |
| Phenoxyethanol, Ethylhexylglycerin | 1.0 | 1.0 | 1.0 | 1.0 | 1.0 | 1.0 | 1.0 |
| Pyridoxin HCl | 0.5 | 0.5 | 0.5 | 0.5 | 0.5 | 0.5 | 0.5 |
| Hyaluronic Acid (Hyasol PF) | | | 3.0 | | 3.0 | | 3.0 |

| Aqua | Ad 100 | | | | | | |
|---|---|---|---|---|---|---|---|
| ΔE values after 6 versus t0 | 6.77 | 4.53 | 4.91 | 5.28 | 5.89 | 4.84 | 4.17 |

**Table 3**

| INCI | Ref. 1 Wt% | Inv. 1 Wt% | Inv. 2 Wt% | Inv. 3 Wt% | Inv. 4 Wt% | Inv. 5 Wt% | Inv. 6 Wt% |
|---|---|---|---|---|---|---|---|
| Potassium Cetyl Phosphate (Amphisol K) | 2.0 | | | | | | |
| PEG-100 Stearate, Glyceryl Stearate (Arlacel 165) | | 2.0 | 2.0 | | | | |
| Steareth-21 (Eumulgin S21) | | | | 2.0 | 2.0 | | |
| PEG-20 Cocoate (Hydriol KP 10) | | | | | | 2.0 | 2.0 |
| Cetyl Alcohol | 3.0 | 3.0 | 3.0 | 3.0 | 3.0 | 3.0 | 3.0 |
| Cetearyl Alcohol | 2.0 | 2.0 | 2.0 | 2.0 | 2.0 | 2.0 | 2.0 |
| Dimethicone | 3.0 | 3.0 | 3.0 | 3.0 | 3.0 | 3.0 | 3.0 |
| Caprylic/Capric Triglyceride | 10.0 | 10.0 | 10.0 | 10.0 | 10.0 | 10.0 | 10.0 |
| Glycerin | 3.0 | 3.0 | 3.0 | 3.0 | 3.0 | 3.0 | 3.0 |
| Xanthan Gum | 0.3 | 0.3 | 0.3 | 0.3 | 0.3 | 0.3 | 0.3 |
| Phenoxyethanol, Ethylhexylglycerin | 1.0 | 1.0 | 1.0 | 1.0 | 1.0 | 1.0 | 1.0 |
| Pyridoxin HCl | 0.1 | 0.1 | 0.1 | 0.1 | 0.1 | 0.1 | 0.1 |
| Hyaluronic Acid (Hyasol PF) | | | 3.0 | | 3.0 | | 3.0 |

| Aqua | Ad 100 | | | | | | |
|---|---|---|---|---|---|---|---|
| ΔE values after 6 weeks storage versus t0 | 3.34 | 2.40 | 2.21 | 2.69 | 1.94 | 1.82 | 1.63 |

Surprisingly, formulas containing PEG-emulsifiers showed lower ΔE values, corresponding to a lower discoloration of the formulations, compared to formulations containing a cetyl phosphate as emulsifier.

## Claims

1. Use of a PEG-emulsifier, optionally in combination with hyaluronic acid or a salt thereof for suppressing discoloration of topical compositions comprising Vitamin B6 or a derivative thereof, wherein the amount of the Vitamin B6 or a derivative thereof in the topical compositions is selected in the range of 0.02 to 6 wt. % and the amount of the PEG-emulsifier is selected in the range of 0.02 to 6 wt.-%, based on the total weight of the composition.

2. The use according to claim 1, wherein the Vitamin B6 or derivative thereof is pyridoxine hydrochloride.

3. The use according to claim 1 or 2, wherein the PEG emulsifier is PEG-100 stearate, optionally in combination with glyceryl stearate, PEG-20 Cocoate and/ or Steareth-21.

4. The use according to claim any one of claims 1 to 3, wherein a hyaluronic acid or a salt thereof is present and wherein the hyaluronic acid or a salt thereof is sodium hyaluronate.

5. The use according to any one of claims 1 to 4, wherein the amount of the Vitamin B6 or a derivative thereof in the topical compositions is selected in the range of 0.05 to 4 wt. %, preferably in the range of 0.1 to 3 wt. %, based on the total weight of the composition.

6. The use according to any one of claims 1 to 5, wherein the amount of the PEG-emulsifier is selected in the range of 0.1 to 3 wt.-%, based on the total weight of the composition.

7. The use according to any one of claims 1 to 6, wherein the amount of the hyaluronic acid or a salt thereof is selected in the range of 0.01 to 5 wt.-%, preferably in the range of 0.02 to 3 wt.-%, most preferably in the range of 0.05 to 2 wt.-%, based on the total weight of the composition.

8. The use according to any one of claims 1 to 7, wherein the topical composition is an O/W emulsion comprising an oily phase dispersed in an aqueous phase in the presence of the PEG-emulsifier.

9. Method for reducing discoloration of topical compositions containing Vitamin B6 or a derivative thereof, comprising:
- preparing a topical composition comprising Vitamin B6 or a derivative thereof, a PEG-emulsifier and optionally hyaluronic acid or a salt thereof and a cosmetically acceptable carrier, wherein the amount of the Vitamin B6 or a derivative thereof in the topical compositions is selected in the range of 0.02 to 6 wt.-% and the amount of the PEG-emulsifier is selected in the range of 0.02 to 6 wt.-%, based on the total weight of the composition; and
- storing the respective composition for at least 2 months.

10. The method according to claim 9 wherein the Vitamin B6 or derivative thereof is pyridoxine hydrochloride.

11. The method according to claim 9 or 10, wherein the PEG-emulsifier is PEG-100 stearate used in combination with glyceryl stearate.

12. The method according to any one of claims 9 to 11, wherein a hyaluronic acid or a salt thereof is present and wherein the hyaluronic acid or a salt thereof is sodium hyaluronate.

13. The method according to any one of claims 9 to 12, wherein the amount of the Vitamin B6 or a derivative thereof in the topical compositions is selected in the range of 0.02 to 6 wt.-%, preferably in the range of 0.05 to 4 wt.-%, most preferably in the range of 0.1 to 3 wt.-%, based on the total weight of the composition.

14. The method according to any one of claims 9 to 13, wherein the amount of the PEG-emulsifier is selected in the range of 0.05 to 4 wt.-%, preferably in the range of 0.1 to 3 wt.-%, based on the total weight of the composition.

15. The method according to any one of claims 9 to 14, wherein the amount of the hyaluronic acid or a salt thereof is selected in the range of 0.02 to 3 wt.-%, preferably in the range of 0.05 to 2 wt.-%, based on the total weight of the composition.

## Patentansprüche

1. Verwendung eines PEG-Emulgators, gegebenenfalls in Kombination mit Hyaluronsäure oder einem Salz davon, zur Unterdrückung von Verfärbungen von Vitamin B6 oder ein Derivat davon umfassenden topischen Zusammensetzungen, wobei die Menge an Vitamin B6 oder einem Derivat davon in den topischen Zusammensetzungen im Bereich von 0,02 bis 6 Gew.-% ausgewählt ist und die Menge an PEG-Emulgator im Bereich von 0,02 bis 6 Gew.-% ausgewählt ist, bezogen auf das Gesamtgewicht der Zusammensetzung.

2. Verwendung nach Anspruch 1, wobei es sich bei dem Vitamin B6 oder dem Derivat davon um Pyridoxinhydrochlorid handelt.

3. Verwendung nach Anspruch 1 oder 2, wobei es sich bei dem PEG-Emulgator um PEG-100-stearat handelt, gegebenenfalls in Kombination mit Glycerylstearat, PEG-20-cocoat und/oder Steareth-21.

4. Verwendung nach einem der Ansprüche 1 bis 3, wobei eine Hyaluronsäure oder ein Salz davon vorhanden ist und wobei es sich bei der Hyaluronsäure oder einem Salz davon um Natriumhyaluronat handelt.

5. Verwendung nach einem der Ansprüche 1 bis 4, wobei die Menge an Vitamin B6 oder einem Derivat davon in den topischen Zusammensetzungen im Bereich von 0,05 bis 4 Gew.-%, vorzugsweise im Bereich von 0,1 bis 3 Gew.-%, ausgewählt ist, bezogen auf das Gesamtgewicht der Zusammensetzung.

6. Verwendung nach einem der Ansprüche 1 bis 5, wobei die Menge an PEG-Emulgator im Bereich von 0,1 bis 3 Gew.-% ausgewählt ist, bezogen auf das Gesamtgewicht der Zusammensetzung.

7. Verwendung nach einem der Ansprüche 1 bis 6, wobei die Menge an Hyaluronsäure oder einem Salz davon im Bereich von 0,01 bis 5 Gew.-%, vorzugsweise im Bereich von 0,02 bis 3 Gew.-%, am meisten bevorzugt im Bereich von 0,05 bis 2 Gew.-%, ausgewählt ist, bezogen auf das Gesamtgewicht der Zusammensetzung.

8. Verwendung nach einem der Ansprüche 1 bis 7, wobei es sich bei der topischen Zusammensetzung um eine O/W-Emulsion handelt, die eine in einer wässrigen Phase in Gegenwart des PEG-Emulgators dispergierte Ölphase umfasst.

9. Verfahren zur Verringerung der Verfärbung von Vitamin B6 oder ein Derivat davon enthaltenden topischen Zusammensetzungen, umfassend:
- die Herstellung einer topischen Zusammensetzung, die Vitamin B6 oder ein Derivat davon, einen PEG-Emulgator und gegebenenfalls Hyaluronsäure oder ein Salz davon und einen kosmetisch unbedenklichen Träger umfasst, wobei die Menge an Vitamin B6 oder einem Derivat davon in den topischen Zusammensetzungen im Bereich von 0,02 bis 6 Gew.-% ausgewählt ist und die Menge an PEG-Emulgator im Bereich von 0,02 bis 6 Gew.-% ausgewählt ist, bezogen auf das Gesamtgewicht der Zusammensetzung, und
- die Lagerung der betreffenden Zusammensetzung für mindestens 2 Monate.

10. Verfahren nach Anspruch 9, wobei es sich bei dem Vitamin B6 oder dem Derivat davon um Pyridoxinhydrochlorid handelt.

11. Verfahren nach Anspruch 9 oder 10, wobei es sich bei dem PEG-Emulgator um PEG-100-stearat handelt, das in Kombination mit Glycerylstearat verwendet wird.

12. Verfahren nach einem der Ansprüche 9 bis 11, wobei eine Hyaluronsäure oder ein Salz davon vorhanden ist und wobei es sich bei der Hyaluronsäure oder einem Salz davon um Natriumhyaluronat handelt.

13. Verfahren nach einem der Ansprüche 9 bis 12, wobei die Menge an Vitamin B6 oder einem Derivat davon in den topischen Zusammensetzungen im Bereich von 0,02 bis 6 Gew.-%, vorzugsweise im Bereich von 0,05 bis 4 Gew.-%, am meisten bevorzugt im Bereich von 0,1 bis 3 Gew.-%, ausgewählt ist, bezogen auf das Gesamtgewicht der Zusammensetzung.

14. Verfahren nach einem der Ansprüche 9 bis 13, wobei die Menge an PEG-Emulgator im Bereich von 0,05 bis 4 Gew.-%, vorzugsweise im Bereich von 0,1 bis 3 Gew.-%, ausgewählt ist, bezogen auf das Gesamtgewicht der Zusammensetzung.

15. Verfahren nach einem der Ansprüche 9 bis 14, wobei die Menge an Hyaluronsäure oder einem Salz davon im Bereich von 0,02 bis 3 Gew.-%, vorzugsweise im Bereich von 0,05 bis 2 Gew.-%, ausgewählt ist, bezogen auf das Gesamtgewicht der Zusammensetzung.

## Revendications

1. Utilisation d'un émulsifiant PEG, éventuellement en combinaison avec de l'acide hyaluronique ou un sel de celui-ci pour supprimer la décoloration de compositions topiques comprenant de la vitamine B6 ou un dérivé de celle-ci, dans laquelle la quantité de vitamine B6 ou d'un dérivé de celle-ci dans les compositions topiques est choisie dans la plage de 0,02 à 6 % en poids et la quantité d'émulsifiant PEG est choisie dans la plage de 0,02 à 6 % en poids, sur la base du poids total de la composition.

2. Utilisation selon la revendication 1, dans laquelle la vitamine B6 ou un dérivé de celle-ci est le chlorhydrate de pyridoxine.

3. Utilisation selon la revendication 1 ou 2, dans laquelle l'émulsifiant PEG est le stéarate de PEG-100, éventuellement en combinaison avec le stéarate de glycéryle, le cocoate de PEG-20 et/ou le stéareth-21.

4. Utilisation selon l'une quelconque des revendications 1 à 3, dans laquelle un acide hyaluronique ou un sel de celui-ci est présent et dans laquelle l'acide hyaluronique ou un sel de celui-ci est l'hyaluronate de sodium.

5. Utilisation selon l'une quelconque des revendications 1 à 4, dans laquelle la quantité de vitamine B6 ou d'un dérivé de celle-ci dans les compositions topiques est choisie dans la plage de 0,05 à 4 % en poids, de préférence dans la plage de 0,1 à 3 % en poids, par rapport au poids total de la composition.

6. Utilisation selon l'une quelconque des revendications 1 à 5, dans laquelle la quantité de l'émulsifiant PEG est choisie dans la plage de 0,1 à 3 % en poids, sur la base du poids total de la composition.

7. Utilisation selon l'une quelconque des revendications 1 à 6, dans laquelle la quantité d'acide hyaluronique ou d' un sel de celui-ci est choisie dans la plage de 0,01 à 5 % en poids, préférablement dans la plage de 0,02 à 3 % en poids, le plus préférablement dans la plage de 0,05 à 2 % en poids, sur la base du poids total de la composition.

8. Utilisation selon l'une quelconque des revendications 1 à 7, dans laquelle la composition topique est une émulsion H/E comprenant une phase huileuse dispersée dans une phase aqueuse en la présence d'un émulsifiant PEG.

9. Procédé pour réduire la décoloration de compositions topiques contenant de la vitamine B6 ou un dérivé de celle-ci, comprenant :
- préparation d'une composition topique comprenant de la vitamine B6 ou un dérivé de celle-ci, un émulsifiant PEG et éventuellement de l'acide hyaluronique ou un sel de celui-ci et un support cosmétiquement acceptable, dans lequel la quantité de vitamine B6 ou un dérivé de celle-ci dans les compositions topiques est choisie dans la plage de 0,02 à 6 % en poids et la quantité de l'émulsifiant PEG est choisie dans la plage de 0,02 à 6 % en poids, par rapport au poids total de la composition ; et
- stockage de la composition respective pendant au moins 2 mois.

10. Procédé selon la revendication 9, dans lequel la vitamine B6 ou un dérivé de celle-ci est le chlorhydrate de pyridoxine.

11. Procédé selon la revendication 9 ou 10, dans lequel l'émulsifiant PEG est le stéarate de PEG-100 utilisé en combinaison avec le stéarate de glycéryle.

12. Procédé selon l'une quelconque des revendications 9 à 11, dans lequel un acide hyaluronique ou un sel de celui-ci est présent et dans lequel l'acide hyaluronique ou un sel de celui-ci est l'hyaluronate de sodium.

13. Procédé selon l'une quelconque des revendications 9 à 12, dans lequel la quantité de vitamine B6 ou d'un dérivé de celle-ci dans les compositions topiques est choisie dans la plage de 0,02 à 6 % en poids, de préférence dans la plage de 0,05 à 4 % en poids, tout particulièrement de préférence dans la plage de 0,1 à 3 % en poids, par rapport au poids total de la composition.

14. Procédé selon l'une quelconque des revendications 9 à 13, dans lequel la quantité de l'émulsifiant PEG est choisie dans la plage de 0,05 à 4 % en poids, de préférence dans la plage de 0,1 à 3 % en poids, par rapport au poids total de la composition.

15. Procédé selon l'une quelconque des revendications 9 à 14, dans lequel la quantité de l'acide hyaluronique ou d'un sel de celui-ci est choisie dans la plage de 0,02 à 3 % en poids, de préférence dans la plage de 0,05 à 2 % en poids, sur la base du poids total de la composition.
